# EUROPÄISCHE PATENTSCHRIFT

(11) **EP 1 339 403 B1**
(45) Veröffentlichungstag und Bekanntmachung des Hinweises auf die Patenterteilung: **08.12.2004**
(21) Anmeldenummer: 01989448.4
(22) Anmeldetag: 09.11.2001
(51) Int. Cl.: A61K 31/35, A61K 31/425, A61K 39/395, A61K 31/44, G01N 33/94

(54) **REPINOTAN-KIT**
REPINOTAN KIT
KIT A BASE DE REPINOTAN

(30) Priorität: 22.11.2000 DE 10058119
(43) Veröffentlichungstag der Anmeldung: 03.09.2003
(73) Patentinhaber: Bayer HealthCare AG, 51368 Leverkusen (DE)
(72) Erfinder: KROLL, Werner, Stamford, CT 06906 (US); ROMBOUT, Ferdinand, NL-6343 CL Klimmen (NL); WEBER, Horst, 53844 Troisdorf (DE); RODRIGUEZ, Maria-Luisa, 40699 Erkrath (DE); SENNHENN, Bernd, 67551 Worms (DE); SCHOHE-LOOP, Rudolf, 42327 Wuppertal (DE)
(86) Internationale Anmeldenummer: PCT/EP2001/012968
(87) Internationale Veröffentlichungsnummer: WO 2002/041881

(56) Entgegenhaltungen:
- EP-A- 0 352 613
- EP-A- 0 749 970
- WO-A-99/26621
- WO-A-99/46591
- DE-A- 19 543 476
- DE-A- 19 754 573

## Beschreibung

Die Erfindung betrifft ein Kit umfassend eine pharmazeutische Zusammensetzung enthaltend Repinotan oder ein physiologisch unbedenkliches Salz von Repinotan, und ein Mittel zur Bestimmung der Konzentration von Repinotan oder seinen Metaboliten in Körperflüssigkeiten, welches nach einem immunologischen Verfahren arbeitet sowie neue pharmazeutische Zusammensetzungen enthaltend Repinotan oder ein physiologisch unbedenkliches Salz von Repinotan, und Verfahren zu deren Herstellung.

Zur akuten Behandlung von neurodegenerativen Erkrankungen wie Schlaganfall und Schädel-Hirn Trauma, die häufig zu neurologischen und funktionalen Langzeitdefiziten bei diesen Patienten führen, ist bisher noch kein Behandlungsprinzip weltweit als wirksam akzeptiert. Außer dem Einsatz von Thrombolytika (beispielsweise t-PA) in den ersten 3 Stunden nach einem ischämischen Schlaganfall bzw. dem Einsatz von Nimodipin bei Patienten mit einer traumatischen Subarachnoidalblutung gibt es noch keine medikamentösen Ansätze, die der pathophysiologischen Kaskade ausreichend Rechnung tragen.

Die EP-A-0 352 613 offenbart 2-[4-({[(2R)-Chroman-2-yl]methyl}amino)butyl]-1,2-benzisothiazol-3(2H)-on 1,1-dioxid (generischer Name: Repinotan) und Repinotan-Salze zur Behandlung von Erkrankungen des zentralen Nervensystems, insbesondere Schlaganfall.

In der DE-A-195 43 476 wird die Eignung von Repinotan und seinen Salzen zur Behandlung von Schädel-Hirn-Tauma beschrieben.

Repinotan hat in mehreren klinischen Studien seine gute Verträglichkeit in gesunden Probanden und Patienten nach einem Schlaganfall oder Schädelhirntrauma gezeigt. Darüber hinaus deuten die vorliegenden klinischen Daten darauf hin, dass die neurologischen und funktionalen Defizite (nach 3 bzw. 6 Monaten je nach Indikation) verbessert werden konnten. Insbesondere bei Patienten, die Blutplasmakonzentrationen im Bereich von ca. 5-20 µg/l erreichten, war das Behandlungsergebnis deutlich besser als bei den Patienten, die Placebo erhalten hatten.

Ein besonders guter Behandlungserfolg lässt sich mit dem erfindungsgemäßen Kit, das eine pharmazeutische Zusammensetzung enthaltend Repinotan oder ein physiologisch unbedenkliches Salz von Repinotan und ein Mittel zur Bestimmung der Konzentration von Repinotan oder seinen Metaboliten in Körperflüssigkeiten welches nach einem immunologischen Verfahren arbeitetumfasst, erzielen.

Der Einsatz des erfindungsgemäßen Kits erlaubt es, ein Arzneimittel, das als Wirkstoff Repinotan in Form der freien Base oder eines Säureadditionssalzes enthält, zu applizieren und innerhalb von kurzer Zeit die aktuellen Konzentrationen von Repinotan oder dessen Metaboliten in Körperflüssigkeiten des behandelten Patienten zu bestimmen. Somit können ohne zeitliche Verzögerung und mit geringem personellem und apparativem Aufwand die für eine optimale Therapie gegebenenfalls erforderlichen Dosisanpassungen vorgenommen werden. Das erfindungsgemäße Kit stellt daher einen wesentlichen Fortschritt in der Akuttherapie neurodegenerativer Erkrankungen insbesondere von Schlaganfall und Schädelhimtrauma dar.

Die vom erfindungsgemäßen Kit umfassten, pharmazeutischen Zusammensetzungen können beispielsweise als Tabletten, Dragees, Pillen, Granulate, Aerosole, Sirupe, Emulsionen, Suspension, Lösungen oder Lyophilisate, die zu einer Lösung rekonstituiert werden können, vorliegen. Die pharmazeutischen Zusammensetzungen enthalten als Wirkstoff Repinotan oder ein physiologisch unbedenkliches Salz von Repinotan. Hierbei soll der Wirkstoff in einer Konzentration von etwa 0,05 bis 95 Gew.-%, bevorzugt von etwa 0,5 bis 90 Gew.-% der Gesamtmischung vorhanden sein, d.h. in Mengen, die ausreichend sind, um den angegebenen Dosierungsspielraum zu erreichen. Neben dem Wirkstoff enthalten die pharmazeutischen Zusammensetzungen zusätzlich inerte, nicht-toxische, pharmazeutisch geeignete Hilfsstoffe.

Repinotan hat folgende Strukturformel: 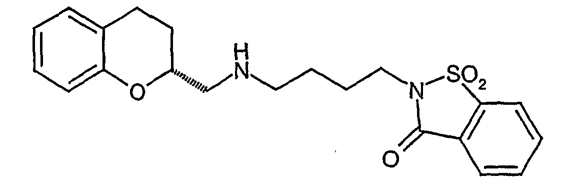 Physiologisch unbedenkliche Salze von Repinotan können Salze von Repinotan mit Mineralsäuren, Carbonsäuren oder Sulfonsäuren sein. Bevorzugt sind Salze von Repinotan mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure. Besonders bevorzugt ist Repinotan Hydrochlorid.

Die Wahl der Hilfsstoffe ist abhängig von der Art der Formulierung. Als Hilfsstoffe erwähnt seien beispielsweise Wasser, nicht-toxische organische Lösungsmittel, wie Paraffine (z.B. Erdölfraktionen), pflanzliche Öle (z.B. Erdnuss/Sesamöl), Alkohole (z.B. Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumsulfat).

Vorzugsweise umfasst das erfindungsgemäße Kit als pharmazeutische Zusammensetzung eine den Wirkstoff enthaltende Infusionslösung oder eine feste pharmazeutische Zusammensetzung, aus der dann diese Infusionslösung durch Zugabe von Wasser oder isotonische Elektrolytlösung hergestellt werden kann.

Eine bevorzugte feste pharmazeutische Formulierung ist das Lyophilisat, das durch Zugabe von Wasser oder einer isotonischen Elektrolytlösung zu beispielsweise einer Infusionslösung rekonstituiert werden kann. Im Lyophilisat hat der Wirkstoff eine erhöhte Lagerstabilität, und aus dem Lyophilisat kann leicht und schnell unter sterilen Bedingungen eine teilchenfreie Lösung hergestellt werden, die direkt zur Anwendung beim Kranken, beispielsweise als Infusionslösung, geeignet ist. Zusätzlich zum Wirkstoff enthält das Lyophilisat vorteilhafterweise weitere pharmazeutisch geeignete Hilfsstoffe, insbesondere Gerüstbildner.

Gerüstbildner im Sinne der Erfindung sind Aminosäuren wie Glycin, Alanin oder Asparaginsäure, Peptide wie Gelatine, Collagen oder Albumin, Monosaccharide wie Glucose oder Lactose, Disaccharide wie Maltose, Saccharose oder Trchalose, Oligosacharide wie Cyclodextrine oder Maltodextrine, Polysaccharide wie Stärke und Stärkederivate oder Cellulose und Cellulosederivate, polymere Gerüstbildner wie Polyvinylpyrrolidon oder Polyethylenglycol, Salze wie Natriumchlorid oder Calciumcarbonat, Zuckeralkohole wie Mannit, Sorbit oder Xylit. Bevorzugt sind Mannit, Kochsalz, Glycin, Saccharose, Maltose oder Lactose. Ganz besonders bevorzugt ist Mannit.

Wenn das Lyophilisat zu einer Infusionslösung rekonstituiert wird, ist diese vorteilhafterweise isotonisch. Dies kann dadurch erreicht werden, dass bereits das Lyophilisat ausreichende Mengen an Elektrolyt, wie beispielsweise Kochsalz, Mannit oder Glucose, enthält, oder dadurch, dass eine isotonische Elektrolytlösung zur Rekonstitution und Verdünnung der Lösung verwendet wird.

Isotonische Elektrolytlösungen sind beispielsweise wässrige 0,9 Gew.-% Kochsalzlösungen oder 5 Gew.-% Glucoselösungen.

Der Anteil des Wirkstoffs an der Infusionslösung sollte etwa 0,1 µg/ml bis 1 mg/ml, bevorzugt etwa 0,5 bis 5 µg/ml betragen.

Die Herstellung von Repinotan und Repinotan-Salzen ist in der EP-A-0 352 613 beschrieben. Repinotan Hydrochlorid entspricht dort dem Beispiel 92H.

Die erfindungsgemäßen Salze können außerdem erhalten werden, indem man die freie Base Repinotan in geeigneten Lösemitteln mit stöchiometrischen oder überstöchiometrischen Mengen der dem Salz zugrundeliegenden Säure in einem Temperaturbereich von 0°C bis zum Siedepunkt des Lösemittels umsetzt. Geeignete Lösemittel sind beispielsweise Wasser, aliphatische Alkohole wie Methanol, Ethanol, oder 2-Propanol, aliphatische offenkettige oder cyclische Ether wie Diethylether, tert.Butyl-methylether, Dioxan, Tetrahydrofuran oder aliphatische Ketone wie 2-Propanon, 2-Butanon, sowie deren Gemische. Die Salze werden direkt aus dieser Mischung, gegebenenfalls nach teilweisem oder vollständigem Abdestillieren des Lösemittels, als Feststoff gewonnen; sie können durch Umkristallisation oder Umfällung in zum Beispiel oben aufgeführten Lösemitteln oder deren Gemischen gereinigt werden.

Die erfindungsgemäßen pharmazeutischen Zusammensetzungen des Kits können auch Gemische aus Repinotan und seinen Salzen oder aus verschiedenen Repinotan-Salzen enthalten.

Repinotan oder seine physiologisch unbedenklichen Salze können in bekannter Weise in die üblichen Formulierungen überführt werden, wie Tabletten, Dragees, Pillen, Granulate, Aerosole, Sirupe, Emulsionen, Suspension und Lösungen, unter Verwendung inerter, nicht toxischer, pharmazeutisch geeigneter Trägerstoffe oder Lösungsmittel. Bevorzugt sind Wirkstofflösungen oder feste pharmazeutische Zusammensetzungen, die in eine Lösung überführt werden können, wie beispielsweise Lyophilisate. Hierbei soll der therapeutisch wirksame Wirkstoff jeweils in einer Konzentration von etwa 0,05 bis 95 Gew.-%, bevorzugt von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Außerdem umfasst das erfindungsgemäße Kit Mittel zur Bestimmung der Konzentration von Repinotan oder seinen Metaboliten in Körperflüssigkeiten.

Es kann vorteilhaft sein, statt des eingesetzten Wirkstoffes als Analyten einen Metaboliten des Repinotans zu bestimmen. Metaboliten des Repinotans sind beschrieben.

Körperflüssigkeiten im Sinne der Erfindung sind beispielsweise Urin, Blut und aus Blut gewonnene Fraktionen wie Serum oder Plasma. Bevorzugt sind Blut und aus Blut gewonnene Fraktionen, besonders bevorzugt Blutplasma.

Die Bestimmung der Konzentration von Repinotan erfolgt in Blut oder in aus Blut gewonnenen Fraktionen, vorzugsweise in Blutplasma.

Als Mittel zur Konzentrationsbestimmung geeignet sind Mittel, die nach immunologischen Methoden arbeiten, wie beispielsweise ELISA's (enzyme linked immunosorbent assays). Mittel dieser Art sind bekannt.

Besonders geeignet sind Mittel, die am Ort der Behandlung eingesetzt werden können (bedside- oder point-of-care-Tests), da mit diesen ohne zeitliche Verzögerung die für eine optimale Therapie gegebenenfalls erforderlichen Dosisanpassungen vorgenommen werden können.

Bevorzugt werden im erfindungsgemäßen Kit als Mittel die in der WO-A-99/46591 offenbarten Vorrichtungen zur Bestimmung der Konzentration von Analyten. Diese Vorrichtungen können nach den dort beschriebenen Verfahren hergestellt und verwendet werden und erlauben, ohne zeitliche Verzögerung und mit geringem personellem und apparativem Aufwand die für eine optimale Therapie gegebenenfalls erforderlichen Dosisanpassungen vorzunehmen.

Besonders eignet sich das erfindungsgemäße Kit zur akuten Behandlung von neurodegenerativen Erkrankungen, insbesondere Schlaganfall oder Schädel-Hirn-Trauma.

Die Applikation der erfindungsgemäßen pharmazeutischen Zusammensetzung erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wässriger Suspensionen können die Wirkstoffe außer den oben genannten Hilfsstoffen mit verschiedenen Geschmacksaufbesserern oder Farbstoffen versetzt werden. Für den Fall der parenteralen Anwendung können Lösungen der Wirkstoffe unter Verwendung geeigneter flüssiger Trägermaterialien eingesetzt werden.

Im allgemeinen hat es sich bei intravenöser Applikation als vorteilhaft erwiesen, den Wirkstoff in Mengen von etwa 0,01 µg/kg/h bis 10 µg/kg/h (µg oder mg pro kg Körpergewicht pro Stunde), vorzugsweise etwa 0,05 µg/kglh bis 2 µg/kg/h zur Erzielung wirksamer Ergebnisse zu verabreichen. Die Verabreichung kann jeweils in Form von Einzelgaben erfolgen.

Bei oraler Applikation beträgt die Tagesdosis des Wirkstoffs 0,001 bis 0,2 mg/kg, vorzugsweise 10 bis 100 µg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Um die Patienten auf die therapeutisch günstigste Konzentration von Repinotan einzustellen, wird den Patienten eine definierte Anfangsdosis von Repinotan verabreicht, nach festen, vorher bestimmten Zeitpunkten die Konzentration von Repinotan mit den erfindungsgemäßen Mitteln kontrolliert und dann die weitere Dosierung gegebenenfalls angepasst.

Beispielsweise werden Patienten mit einer Lösung, die Repinotan Hydrochlorid enthält, mit einer Rate von 1,25 mg Repinotan/Tag infundiert. Nach 6 Stunden wird mit dem erfindungsgemäßen Mittel bestimmt, ob die Repinotan-Plasmakonzentration über oder unter ca. 17 µg/l liegt. Wenn die Repinotan-Plasmakonzentration unter der angegebenen Grenzkonzentration liegt, wird die Infusion mit der angegebenen Rate weitergeführt. Übersteigt dagegen die Repinotan-Plasmakonzentration die angegebene Grenzkonzentration, wird die Infusionsrate auf 0,5 mg Repinotan/Tag reduziert. 12 Stunden nach Infusionsbeginn wird die Repinotan-Plasmakonzentration ein weiteres Mal kontrolliert. Wiederum wird die Infusionsrate beibehalten, wenn die Repinotan-Plasmakonzentration unter ca. 17 µg/l liegt, und auf 0,625 bzw. 0,25 mg Repinotan/ Tag reduziert, wenn die Repinotan-Plasmakonzentration ca. 17 µg/l übersteigt. Auf diese Weise gelingt es bei einer maximalen Anzahl von Patienten, die Repinotan-Plasmakonzentration im günstigen therapeutischen Dosisbereich von ca. 5 bis 20 µg/l zu halten.

In einem weiteren Aspekt betrifft die Erfindung feste pharmazeutische Zusammensetzungen enthaltend Repinotan oder dessen physiologisch verträgliches Salz, und eine physiologisch unbedenkliche Säure.

Überraschenderweise zeigt sich, dass durch Zusatz einer physiologisch unbedenklichen Säure die Lagerstabilität fester pharmazeutischer Zusammensetzungen enthaltend Repinotan oder dessen physiologisch verträglicher Salze erhöht wird.

Physiologisch unbedenkliche Säuren im Sinne der Erfindung sind beispielsweise Äpfelsäure, Asparaginsäure, Ascorbinsäure, Benzoesäure, Bernsteinsäure, Brenztraubensäure, Citronensäure, Fumarsäure, Glutaminsäure, Glycolsäure, Hippursäure, Maleinsäure, Milchsäure, Schwefelsäure, Sorbinsäure, Weinsäure oder Zimtsäure. Bevorzugt sind Äpfelsäure, Brenztraubensäure, Citronensäure, Fumarsäure, Maleinsäure, Milchsäure und Weinsäure. Besonders bevorzugt sind Citronensäure und Weinsäure.

Die erfindungsgemäße Wirkung wird insbesondere mit festen pharmazeutischen Zusammensetzungen erreicht, bei denen der Wirkstoff, d.h. Repinotan oder dessen physiologisch verträglicher Salze, 0,01 - 15 Gew.-% und die physiologisch unbedenklichen Säure 0,5 - 15 Gew.-% des Gesamtgewichtes der Zusammensetzung ausmachen.

Ganz besonders bevorzugt sind dabei feste pharmazeutische Zusammensetzungen enthaltend Repinotan Hydrochlorid und Citronensäure oder Weinsäure.

Die erfindungsgemäßen festen pharmazeutischen Zusammensetzungen können nach üblichen Verfahren hergestellt werden.

Ein weiterer Aspekt der Erfindung betrifft Lyophilisate enthaltend Repinotan oder dessen physiologisch verträgliche Salze, und eine physiologisch unbedenkliche Säure, wobei als Wirkstoff Repinotan Hydrochlorid, als Säuren Äpfelsäure, Brenztraubensäure, Citronensäure, Fumarsäure, Maleinsäure, Milchsäure und Weinsäure, insbesondere Citronensäure und Weinsäure, bevorzugt sind. Zusätzlich können die Lyophilisate vorteilhafterweise pharmazeutische Hilfsstoffe wie beispielsweise die oben genannten Gerüstbildner enthalten.

Zur Herstellung des Lyophilisats werden beispielsweise die oben genannten Mengen des Repinotans oder Repinotan-Salzes, der physiologisch unbedenklichen Säure und gegebenenfalls weitere pharmazeutische Hilfsstoffe in Wasser und/oder anderen geeigneten Lösungsmitteln gelöst, steril gemacht, in geeignete Behälter gefüllt und gefriergetrocknet.

Geeignete Lösungsmittel im Sinne der Erfindung sind beispielsweise Eisessig oder tert.-Butanol.

Die Lösung kann üblicherweise bei Temperaturen von 5 bis 35°C hergestellt werden, bevorzugt bei 20 bis 25°C. Sterilmachen der Lösung im Sinne der Erfindung bedeutet die Überführung der Lösung in den sterilen Zustand, beispielsweise durch Sterilfiltration durch Filter mit maximaler Porenweite von 0,2 µm aus geeigneten Filtermaterialien wie z.B. Polyethersulfon oder Nylon 6.6.

Geeignete Behälter für die Gefriertrocknung sind beispielsweise Hüttenglas- oder Röhrenglasflaschen. Das Abfüllvolumen der Lösung beträgt zwischen 0,2 ml und 20 ml, bevorzugt 0,5 ml bis 5 ml, besonders bevorzugt 0,7 ml bis 3,0 ml.

Die Lösung wird in den Behältern gefriergetrocknet. Dazu werden die Behälter mit der Lösung auf vorgekühlte oder nicht vorgekühlte Stellplatten gestellt und eingefroren. Die anschließende Haupttrocknung wird im Vakuum bei Kammerdrucken von 0,05 mbar bis 1,8 mbar mit Stellplattentemperaturen von -40°C bis +60°C durchgeführt. Die Nachtrocknung wird bei Kammerdrucken von 1 µbar bis 1,8 mbar mit Stellplattentemperaturen von -20°C bis +70°C durchgeführt.

Alternativ kann das Gefriertrocknungsverfahren nach dem in der internationalen Anmeldung PCT/EP00/07034 beschriebenen Verfahren durchgeführt werden. Durch die Gefriertrocknung entsteht das lagerstabile Lyophilisat der oben genannten Zusammensetzungen.

Außerdem betrifft die Erfindung Infusionslösungen enthaltend Repinotan oder ein physiologisch unbedenkliches Salz von Repinotan, und eine oder mehrere Säuren ausgewählt aus der Gruppe Äpfelsäure, Brenztraubensäure, Citronensäure, Fumarsäure, Maleinsäure, Milchsäure und Weinsäure, vorzugsweise Citronensäure oder Weinsäure.

Die erfindungsgemäßen Infusionslösungen zeigen eine unerwartet hohe Lagerstabilität.

Eine Infusionslösung im Sinne der Erfindung ist eine Lösung, die als Lösungsmittel überwiegend Wasser enthält und normalerweise über eine Osmolalität im Bereich von 250 bis 500 mOsmol/kg, bevorzugt 280 bis 350 mOsmol/kg verfügt. Die oben genannte Osmolalität der Infusionslösung kann dadurch erzielt werden, dass die notwendigen Mengen an beispielsweise Kochsalz, Mannit oder Glucose bereits Bestandteil der festen pharmazeutischen Zusammensetzung sind und mit Wasser rekonstituiert und/oder verdünnt wird, oder dass die notwendigen Mengen an diesen Substanzen mit dem Rekonstitutions-/Verdünnungsmedium zugegeben werden. Die Repinotan-Konzentration der Infusionslösung beträgt gewöhnlich 0,1 bis 500 µg Repinotan/ml Lösung, vorzugsweise 0,5 bis 5µg/ml. Die Infusionslösung enthält soviel Säure, dass bei 20°C der pH-Wert 3,5 bis 5, vorzugsweise ungefähr 4 beträgt.

Normalerweise erfolgt die Zugabe dieser Lösungen bei Raumtemperatur.

### Ausführungsbeispiele:

### Beispiel 1: Lyophilisat einer Zusammensetzung enthaltend Repinotan Hydrochlorid

Eine Mischung aus 55 mg Repinotan Hydrochlorid (entspricht 50 mg Repinotan), 20 g Mannit und 3,7 g Citronensäure wurde auf 1000 ml mit Wasser aufgefüllt. Diese wässrige Lösung wurde über Filter mit einer maximalen Porenweite von 0,2 µm aus Polyethersulfon sterilfiltriert und in Röhrenglasflaschen abgefüllt. Das Abfüllvolumen der Lösung betrug 1 ml.

Die Lösung wurde in den Röhrenglasflaschen gefriergetrocknet. Dazu wurden die Röhrenglasflaschen mit der Lösung bei Raumtemperatur auf die Stellplatten des Gefriertrockners gestellt und bei -40°C eingefroren. Die anschließende Haupttrocknung wurde im Vakuum bei einem Kammerdruck von 0,2 mbar bei einer Stellplattentemperatur von 0°C durchgeführt. Die Nachtrocknung wurde bei einem Kammerdruck von 10 µbar mit einer Stellplattentemperatur von 40°C durchgeführt. Durch die Gefriertrocknung entsteht das lagerstabile Endprodukt.

Alternativ kann die Gefriertrocknung nach dem in der internationalen Anmeldung PCT/EP00/07034 beschriebenen Verfahren durchgeführt werden.

Analog dem in Beispiel 1 beschriebenen Verfahren wurden mit den in Beispiel 1 benutzten Mengen von Mannitol, Citronensäure und Wasser Lyophilisate mit 0,27 g, 0,681 g, 1,36 g und 2,73 g Repinotan Hydrochlorid, entsprechend 0,25 g, 0,625 g, 1,25 g bzw. 2,5 g Repinotan, hergestellt.

### Beispiel 2: Infusionslösung enthaltend Repinotan Hydrochlorid

Aus dem gefriergetrockneten Produkt gemäß Beispiel 1 wird eine anwendungsfertige Infusionslösung hergestellt, indem das Lyophilisat mit Hilfe einer 0,9 Gew.-% NaCl-Lösung rekonstituiert und dann mit dieser Lösung auf ein Gesamtvolumen von 500 ml verdünnt wird. Die anwendungsfertige Lösung ist chemisch für mindestens 30 h bei Raumtemperatur lagerstabil, d.h. die Lösung enthält dann noch mindestens 90 % unverändertes Repinotan.

### Beispiel 3: Bestimmung von Repinotan mit Hilfe eines ELISA aufMikrotiterplatte

Der Test wird auf Basis eines kompetitiven ELISA durchgeführt. Das Proteinkonjugat eines dem Repinotan analogen Haptens wird in konstanter Menge mit einer konstanten Menge monoklonalen oder polyklonalen Antikörpers Fab oder Fab2 Fragmentes, spezifisch gegen die chemische Struktur des Repinotan, und einem konstanten Volumen an Repinotan-haltiger Probe zur Reaktion gebracht. Aus dem Verhältnis an gebundenem zu freiem Proteinkonjugat kann dann über eine parallel zu erstellende Eichkurve mit bekannten Konzentrationen an Repinotan in der zu untersuchenden Probe die Konzentration von Repinotan bestimmt werden.

Ziegen-IgG wird mit dem unten genannten Repinotan-Analogen im molaren Verhältnis 1 : 7.5 chemisch verknüpft. Dieses Konjugat wird in einem 0.1 m Natriumcarbonat-Puffer pH=9.6 für 1 h bei 37 oC in einer Microtiterplatte (z. B. Dynex Immulon 2HB) inkubiert. Anschließend wir 30 min bei Raumtemperatur mit einer phosphatgepufferten, caseinhaltigen (1%) Salinelösung geblockt. Nachfolgend wird mit tweenhaltiger (0.05 %) phosphatgepufferter Salinelösung drei Mal gewaschen.

Auf der so vorbereiteten Platte kann nun der kompetitive Test durchgeführt werden. Dazu werden 100 µl Probe in ein Microtiterwell vorgelegt und mit 100 µl Antikörperlösung (maus-anti-Repinotan-Antikörper 10 ng/ml in phosphatgepufferter Salinelösung) versetzt. Bei Raumtemperatur wird 1 h inkubiert. Nach dreimaligem Waschen mit Waschpuffer (s. oben) wird in einer weiteren Reaktion sekundärer Antikörper, gekoppelt an Horseradish Peroxidase (1:1; Zymed Ziege-anti-Maus IgG; 1:1000 verdünnt), in das Microtiterwell pipettiert. Dies bindet an den Repinotan-spezifischen Mausantikörper, der an das Repinotan-analoge Haptenkonjugat gebunden ist. Die Menge an so gebundenem Repinotan-spezifischen Mausantikörper ist der Menge an freiem Repinotan in der Probe indirekt proportional. Durch dreimaliges Waschen wird überschüssiges Antikörper-Enzymkonjugat ausgewaschen und nach Zugabe von Enzymsubstrat (TMB) die verbliebene gebundene Menge photometrisch über eine kinetische Messung bestimmt. Mit dem aufgezeigtem Protokoll lassen sich unter Verwendung entsprechender Kalibratoren Repinotankonzentrationen zwischen 1 und 100 ng/ml bestimmen.

### Beispiel 4: Point-of-Care Repinotan-Test

Dieser Repinotantest ist bestimmt zur Verwendung im Bereich der Intensivpflege und anderen Krankenhausbereichen zur Bestimmung von Repinotankonzentrationen oberhalb und unterhalb einer definierten Grenzkonzentration von ungefähr 17 ng/ml in Vollblut, Serum oder Plasma. Hierbei handelt es sich um einen Einmaltest mit visuellem Readout.

### Beschreibung der Vorrichtung:

Dem Test liegt das oben beschriebene kompetitive immunologische Verfahren zur Bestimmung kleiner Moleküle zugrunde. Als Auslesemechanismus wird anstatt eines enzymatischen spektroskopischen Verfahrens das reflektorische Signal von kolloidalen Goldpartikeln verwendet. Alle notwendigen Testkomponenten und mechanischen Manipulationen, die zur Durchführung konventioneller Immunoassays notwendig sind, sind in einem Plastikdevice untergebracht. Dies beinhaltet einen Mechanismus zur Separation der Blutzellen vom Serum und einen Mechanismus zur exakten Abmessung einer bestimmten Menge des so separierten Serums. Ein Pufferreservoir beinhaltet die für die Reaktion notwendige flüssige Phase, die die Kapillarbewegung innerhalb des Systems ermöglicht. Anti-Repinotan-Antikörper und Hapten-Ziegen-IgG-Gold-Konjugat sind in einer Reaktionszelle des Plastikdevices lyophillisiert. Eine Mischkammer am Ende einer Kapillare sowie ein immunochromatographischer Teststreifen vervollständigen das System. Auf den Teststreifen sind stationäre Reaktionszonen aufgetragen: ein Esel-anti-Maus Antikörper und ein Kaninchen-anti-Ziege-Antikörper.

In Abwesenheit von Repinotan fängt der Esel-anti-Maus Antikörper den überwiegenden Teil des Goldkonjugats ab und bildet eine rote Farbzone. Mit steigender Repinotankonzentration in der zu untersuchenden Probe wird das proportional am anti-Repinotan-Antikörper verdrängte Goldkonjugat in der Kaninchen-anti-Ziege Reaktion abgefangen.

Am Ende des immunochromatographischen Teststreifens ist eine Absorptionszone an deren Beginn ein wasserlöslicher Farbstoff aufgetragen ist. Dieser Farbstoff bewegt sich mit der Flüssigkeitsfront vorwärts und bewegt ihn an das Ende der Absorptionszone. Dies zeigt das Ende der Testreaktionen an.

Die Dauer des Testes beträgt ca. 15 min, von der Applikation der Probe bis zum Auslesen des Ergebnisses. Der Test kann mit 100 - 150 µl Vollblut durchgeführt werden, was die Generierung von ca. 15 µl Serum garantiert.

Die folgenden Reaktions-Komponenten werden zur Herstellung des Testdevices benötigt:
a) Monoclonaler anti-Repinotan
b) Gold-Repinotananalog-Ziegen-IgG-Konjugat
c) kolloidales Gold, 20 nm
d) Nitrocellulose Membrane
e) Esel anti-Maus
f) Kaninchen anti-Ziege
g) Repinotan Standard
h) 0.2% Triton x 305 in Phosphate Buffered Saline Assay Buffer
i) 0.4% Nonidet P-40, 2% BSA, 2.5% Sucrose, 1.25%Trehelose, in Phosphate Buffered Saline Lyophilized Buffer
j) 2.5 cm Sink Material
k) Plastic Backing, 5.3 cm wide and 8 mil. Thick
I) Human Serum

### Testdurchführung:

Ca. 150 µl Vollblut (5 Tropfen), Serum oder Plasma werden auf den Probenteller appliziert. Das Device wird ca. 3 Minuten in horizontaler Position gelagert. Dann wird der Griff des Dosierkolbens um 90° gedreht, bis er flach auf dem Boden des Devices aufliegt. Das Device wird anschließend für 15 min bei Raumtemperatur in horizontaler Position gelagert. Wenn am Indikatorfenster ein blauer Punkt erscheint, ist der Test abgeschlossen und das Ergebnis kann ausgelesen werden.

Außer der Probenauftragung und dem Betätigen des Dosierkolbens sind keine weiteren Manipulationen der Probe oder des Devices zur gesamten Durchführung des Testes notwendig. Der Test kann daher auch von nichttechnischem Personal durchgeführt werden.

### Ergebnisinterpretation:

Wie in der WO-A-99/46591 beschrieben, lässt der Vergleich der Intensität der beiden Banden in den Fenstern A und B die einfache halbquantitative Bestimmung der Repinotankonzentration zu. Ist das Signal in Fenster A intensiver als das in Fenster B, liegt die Repinotankonzentration oberhalb eines bestimmten Grenzwertes, bei umgekehrter Intensitätsverteilung unterhalb eines bestimmten Grenzwertes. Der Grenzwert kann durch das Verhältnis der anti-Repinotan-Antikörperkonzentration und der Konzentration des Gold-Repinotananalogen-Ziegen-IgGs im Lyophilisat festgelegt werden.

Das Repinotananalog im Gold-Repinotananalog-Ziegen-IgG-Konjugat ist eine Verbindung der Formel:

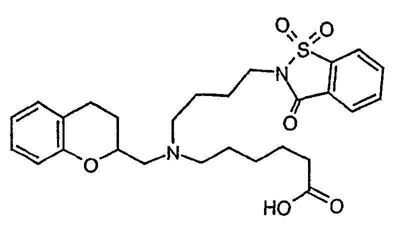

Diese Verbindung ist neu und kann nach den in der EP-A-0 352 613 beschriebenen Verfahren, beispielsweise durch Reaktion von Repinotan mit 6-Bromo-hexancarbonsäure Tertiärbutylester in Gegenwart einer Base in geeigneten Lösungsmitteln und anschließender saurer Esterspaltung hergestellt.

## Patentansprüche

1. Kit umfassend
a) eine pharmazeutische Zusammensetzung enthaltend Repinotan und/oder ein physiologisch unbedenkliches Salz von Repinotan, und
b) ein Mittel zur Bestimmung der Konzentration von Repinotan oder seinen Metaboliten in Körperflüssigkeiten, welches nach einem immunologischen Verfahren arbeitet.

2. Kit nach Anspruch 1, wobei die pharmazeutische Zusammensetzung Repinotan Hydrochlorid enthält.

3. Kit nach Anspruch 1 oder 2, wobei das Kit ein Mittel zur Konzentrationsbestimmung des Analyten in Blut oder in aus Blut gewonnenen Fraktionen umfasst.

4. Kit nach Anspruch 3, wobei das Kit ein Mittel zur Konzentrationsbestimmung des Analyten in Blutplasma umfasst.

5. Kit nach einem der Ansprüche 1 bis 4, wobei das Mittel am Ort der Behandlung eingesetzt werden kann.

6. Kit nach einem der Ansprüche 1 bis 5 zur akuten Behandlung von neurodegenerativen Erkrankungen.

7. Kit nach einem der Ansprüche 1 bis 6 zur akuten Behandlung von Schlaganfall oder Schädel-Hirn-Trauma.

8. Feste pharmazeutische Zusammensetzung enthaltend Repinotan oder ein physiologisch unbedenkliches Salz von Repinotan, und eine physiologisch unbedenkliche Säure, wobei der Gewichtsanteil des Wirkstoffs 0,01 - 15 Gew.-%.

9. Zusammensetzung nach Anspruch 8, wobei die Zusammensetzung als Wirkstoff Repinotan Hydrochlorid und als Säure Citronensäure oder Weinsäure enthält.

10. Verfahren zur Herstellung einer Zusammensetzung nach einem der Ansprüche 8 und 9, wobei der Wirkstoff, die Säure und weitere pharmazeutische Hilfsstoffe in Wasser und/oder anderen geeigneten Lösungsmitteln aufgelöst werden, die Lösung gegebenenfalls steril gemacht wird und die Lösung dann lyophilisiert wird.

11. Zusammensetzung erhältlich nach dem Verfahren gemäß Anspruch 10.

12. Verfahren zur Herstellung einer Infusionslösung enthaltend Repinotan oder ein physiologisch unbedenkliches Salz von Repinotan, und Citronensäure, und/oder Weinsäure, wobei zu einer Zusammensetzung gemäß Anspruch 11 Wasser oder eine isotonische Elektrolytlösung gegeben wird.

13. Infusionslösung erhältlich nach dem Verfahren gemäß Anspruch 12.

14. Verwendung von Zusammensetzungen nach Anspruch 11 zur Herstellung von Infusionslösungen.

15. Verbindung der Formel

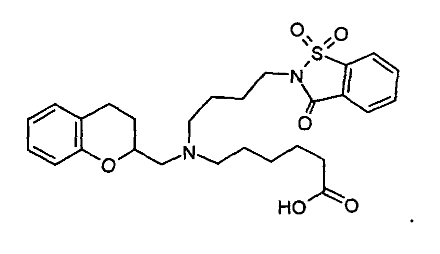

## Claims

1. Kit comprising
a) a pharmaceutical composition containing repinotan and/or a physiologically acceptable salt of repinotan, and
b) a means for the determination of the concentration of repinotan or its metabolites in body fluids, which operates according to an immunological process.

2. Kit according to Claim 1, where the pharmaceutical composition contains repinotan hydrochloride.

3. Kit according to Claim 1 or 2, where the kit comprises a means for determination of the concentration of the analyte in blood or in fractions obtained from blood.

4. Kit according to Claim 3, where the kit comprises a means for determination of the concentration of the analyte in blood plasma.

5. Kit according to one of Claims 1 to 4, where the means can be employed at the site of treatment.

6. Kit according to one of Claims 1 to 5 for the acute treatment of neurodegenerative diseases.

7. Kit according to one of claims 1 to 6 for the acute treatment of stroke or craniocerebral trauma.

8. Solid pharmaceutical composition containing repinotan or a physiologically acceptable salt of repinotan, and a physiologically acceptable acid, where the proportion by weight of the active compound is 0.01-15% by weight and the proportion by weight of the acid is 0.5-15% by weight.

9. Composition according to Claim 8, where the composition contains repinotan hydrochloride as the active compound and citric acid or tartaric acid as the acid.

10. Process for the preparation of a composition according to either of Claims 8 and 9, where the active compound, the acid and further pharmaceutical excipients are dissolved in water and/or other suitable solvents, the solution is optionally rendered sterile and the solution is then lyophilized.

11. Composition obtainable according to the process as in Claim 10.

12. Process for the preparation of an infusion solution containing repinotan or a physiologically acceptable salt of repinotan, and citric acid and/or tartaric acid, where water or an isotonic electrolyte solution is added to a composition as in Claim 11.

13. Infusion solution obtainable according to the process as in Claim 12.

14. Use of compositions according to Claim 11 for the preparation of infusion solutions.

15. Compound of the formula

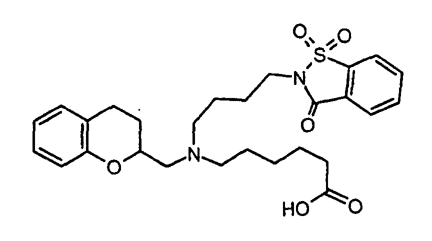

## Revendications

1. Nécessaire comprenant
a) une composition pharmaceutique contenant du répinotan et/ou un sel de répinotan acceptable du point de vue physiologique, et
b) un moyen de détermination de la concentration de répinotan ou de ses métabolites dans des liquides corporels, qui opère selon une méthode immunologique.

2. Nécessaire suivant la revendication 1, dans lequel la composition pharmaceutique contient du chlorhydrate de répinotan.

3. Nécessaire suivant la revendication 1 ou 2, qui comprend un moyen de détermination de la concentration de l'analyte dans le sang ou dans des fractions obtenues à partir de sang.

4. Nécessaire suivant la revendication 3, qui comprend un moyen de détermination de la concentration de l'analyte dans le plasma sanguin.

5. Nécessaire suivant l'une des revendications 1 à 4, dans lequel le moyen peut être utilisé sur le site de traitement.

6. Nécessaire suivant l'une des revendications 1 à 5, destiné au traitement en urgence de maladies neurodégénératives.

7. Nécessaire suivant l'une des revendications 1 à 6, destiné au traitement en urgence de l'apoplexie cérébrale d'un traumatisme craniocérébral.

8. Composition pharmaceutique solide contenant du répinotan ou un sel de répinotan acceptable du point de vue physiologique, et un acide acceptable du point de vue physiologique, la proportion en poids de substances actives étant de 0,01 à 15 % en poids.

9. Composition suivant la revendication 8, qui contient comme substance active du chlorhydrate de répinotan et comme acide, de l'acide citrique ou de l'acide tartrique.

10. Procédé de préparation d'une composition suivant l'une des revendications 8 et 9, dans lequel la substance active, l'acide et d'autres substances pharmaceutiques auxiliaires sont dissous dans l'eau et/ou dans d'autres solvants convenables, la solution est éventuellement stérilisée et elle est ensuite lyophilisée.

11. Composition pouvant être obtenue par le procédé suivant la revendication 10.

12. Procédé de préparation d'une solution pour perfusion contenant du répinotan ou un sel de répinotan acceptable du point de vue physiologique, et de l'acide citrique et/ou de l'acide tartrique, dans lequel de l'eau ou une solution isotonique d'électrolyte est ajoutée à une composition suivant la revendication 11.

13. Solution pour perfusion pouvant être obtenue par le procédé suivant la revendication 12.

14. Utilisation de compositions suivant la revendication 11 pour la préparation de solutions pour perfusion.

15. Composé de formule
